# EUROPEAN PATENT APPLICATION

(11) **EP 3 734 602 A1**
(43) Date of publication of application: **04.11.2020**
(21) Application number: 18897229.3
(22) Date of filing: 14.12.2018
(51) Int. Cl.: G16B 15/00

(54) **WHOLE GENOME SGRNA LIBRARY CONSTRUCTING SYSTEM AND APPLICATION THEREOF**

(30) Priority: 29.12.2017 CN 201711481306
(71) Applicant: Genewiz. Inc Suzhou, Suzhou, Jiangsu 215123 (CN)
(72) Inventor: XU, Fengdan, Suzhou, Jiangsu 215123 (CN); JIN, Liang, Suzhou, Jiangsu 215123 (CN); XU, Pengyang, Suzhou, Jiangsu 215123 (CN); DUAN, Guangyou, Suzhou, Jiangsu 215123 (CN); ZHAO, Wenyan, Suzhou, Jiangsu 215123 (CN); GE, Yi, Suzhou, Jiangsu 215123 (CN)
(74) Representative: Mewburn Ellis LLP
(86) International application number: PCT/CN2018/121328
(87) International publication number: WO 2019/128744

(57) **Abstract**

Provided are a system for constructing a genome-wide sgRNA library and a use thereof. The system includes an input module, an sgRNA design module and an sgRNA filtering module. By constructing three modules in the system, optimizing details and processes in the modules, and adopting multiple design criteria and screening principles, the genome-wide sgRNA library is finally constructed. The system and method are concise and efficient, and the obtained library has a high quality and good activity, and is convenient for applications in gene editing researches.

## Description

### CROSS-REFERENCES TO RELATED APPLICATIONS

This application claims priority to Chinese patent application No. CN201711481306.3 filed with the CNIPA on December 29, 2017 and entitled "Establishment system and application of whole genome sgRNA library", disclosure of which is incorporated herein by reference in its entirety.

### TECHNICAL FIELD

The present application relates to the field of genetic engineering and, in particular, to a system for constructing a genome-wide sgRNA library and a use thereof.

### BACKGROUND

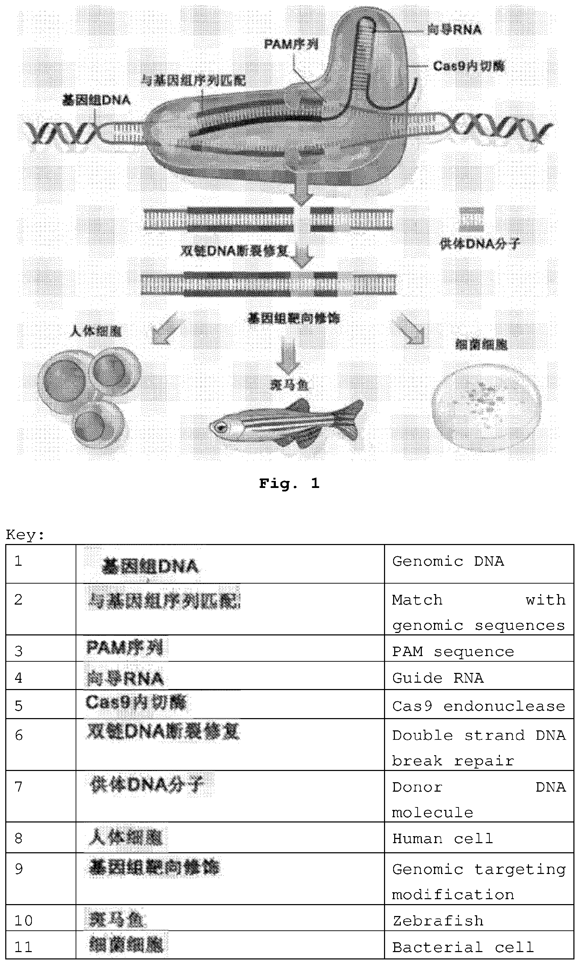

Clustered regularly interspaced short palindromic repeats (CRISPR) are in fact a kind of gene editor, a system of bacteria for protecting themselves against viruses, and also a genetic weapon against an attacker. Later, researchers discovered that the CRISPR seem to be an accurate universal genetic weapon that can be used for deleting, adding, activating or inhibiting target genes of other organisms, including genes in cells of humans, mice, zebrafish, bacteria, fruit flies, yeast, nematodes and crops, which also means that the gene editor is a widely applicable biotechnology. A working process of the CRISPR gene editor is shown in FIG. 1.

A CRISPR cluster is a special family of repeated DNA sequences that are widely present in genomes of bacteria and archaea. Its sequence is composed of a leader, multiple short and highly-conserved repeats and multiple spacers. The leader is generally located upstream of the CRISPR cluster, a region rich in AT with a length of 300-500 bp, and regarded as a promoter sequence of the CRISPR cluster. The repeat has a length of 21-48 bp, contains a palindromic sequence, and can form a hairpin structure. The repeats are spaced by the spacer with a length of 26-72 bp. The spacer is composed of captured foreign DNA and similar to an immune memory. When foreign DNA containing a same sequence invades, the foreign DNA can be recognized by a bacterium body and cleaved to silence its expression, so as to protect the safety of the bacterium body.

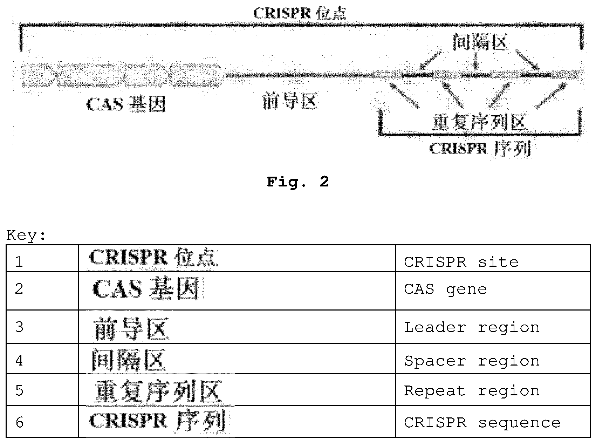

An analysis on flanking sequences of the CRISPR cluster revealed that a family of polymorphic genes exits nearby. Proteins encoded by the family all contain functional domains (with activity of nuclease, helicase, integrase and polymerase) that can interact with nucleic acids and work in collaboration with a CRISPR region. Therefore, the family of polymorphic genes is referred to as CRISPR associated (Cas) genes. Currently discovered Cas includes various types such as Cas1 to Cas10. The Cas genes and the CRISPR evolve together to form a highly-conserved system. A system structure of the CRISPR cluster is shown in FIG. 2.

When bacteria defend against the invasion of foreign DNA such as phages, under the control of the leader, the CRISPR is transcribed into a long RNA precursor (pre RISPR RNA (pre-crRNA)), then processed into a series of short mature crRNAs containing conserved repeated sequences and spacers, and recognized and bound to a complementary foreign DNA sequence to play a cleavage role.

It is currently discovered that a CRISPR/Cas system includes three different types, i.e., type I, type II and type III, which are present in approximately 40% of sequenced true bacteria and 90% of sequenced archaea. Type II has a relatively simple composition with Cas9 protein and a guide RNA (gRNA) as the core, and is most deeply studied at present.

In a type II system, the pre-crRNA is processed solely by Cas9 in the Cas family. Cas9 contains RuvC at an amino terminus and HNH2 unique active sites in the middle of protein and plays a role in crRNA maturation and double-stranded DNA cleavage. In addition, while the pre-crRNA is transcribed, a trans-activating crRNA (tracrRNA) complementary to repeated sequences of the pre-crRNA is also transcribed, and Cas9 and double-stranded RNA-specific nuclease RNase III are stimulated to process the pre-crRNA. After processing and maturation, the crRNA, the tracrRNA and Cas9 form a complex that recognizes and is bound to a complementary sequence of the crRNA, and DNA double strands are unwound to form an R-loop, such that the crRNA is hybridized with a complementary strand and the other strand remains in a free single-strand state. A complementary DNA strand of the crRNA is cleaved by HNH active sites in Cas9, a non-complementary strand is cleaved by an RuvC active site, and finally a DNA double-strand break (DSB) is introduced. A cleavage site of the CRISPR/Cas9 is located at an NGG site in a 5'-GG-N18-NGG-3' characteristic region of a protospacer adjacent motif (PAM) region adjacent to the downstream of the complementary sequence of the crRNA, and a sequence with this characteristic is repeated once in each random DNA sequence of 128bp. Research results showed that Cas9 can also cleave linear and supercoiled plasmids with cleavage efficiency comparable to a restriction enzyme. Since the crRNA participates and plays an accurate guiding role, the CRISPR/Cas9 targeting system is also referred to as an RNA guided targeting system.

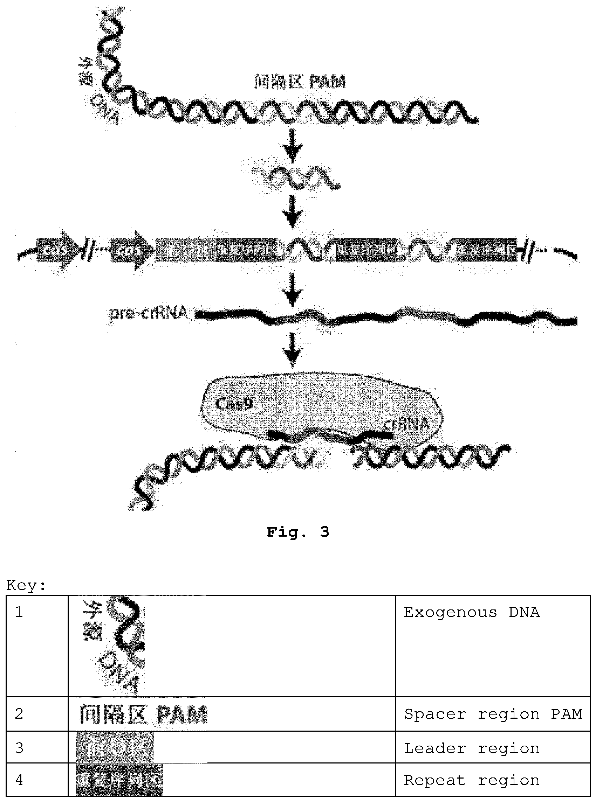

A principle of the targeting system is shown in FIG. 3.

A DNA editing system based on a CRISPR/Cas9-sgRNA has developed into an effective tool for editing genes. A CRISPR/Cas9-sgRNA system includes two main components: Cas9 protein and sgRNA. The sgRNA determines a gene editing site and gene editing efficiency. Researches have shown that different sgRNAs have different editing efficiency. Through high-throughput comparison and analysis of the efficiency of sgRNAs in animals and humans, researchers have obtained parameters of an efficient sgRNA.

CN106845151A has disclosed a method for screening sgRNA targets in a CRISPR-Cas9 system, which includes: (1) acquiring segments with a 5'-Nx-NGG-3' sequence in a genome (where x is an integer between 19 to 22 and N represents A/T/C/G) as candidate targets of an sgRNA in the CRISPR-Cas9 system by using genome-wide sequences and gene annotation information of published species; (2) breaking the genome into segments of 22-25 bp and screening sequences ending with NGG and having no repeats in the genome; (3) comparing sequences of the candidate targets in step (1) with the sequences screened in step (2), and screening and ordering preferred sequences according to mismatch information and a selection formula, to obtain a best set of genome-wide sgRNA targets. CN105886616A has disclosed an efficient and specific sgRNA recognition site guiding sequence for pig gene editing and a screening method thereof. The screening method includes the following steps: functional gene screening and an ORF analysis, functional gene sgRNA recognition site guiding sequence prediction, genome-wide off-target site detection, scoring predicted target sites based on off-target information and positions of target sites, ordering, result screening and statistics, and algorithm optimization and software development. A pig-specific sgRNA recognition site guiding sequence of the present application has undergone rigorous screening and testing and includes all sgRNA recognition site guiding sequences of pig protein-encoding genes for CRISPR-Cas9 gene editing. However, in the existing art, construction steps are cumbersome, optimization and screening criteria are backward, and the obtained sgRNAs are not high in quality.

However, low-quality sgRNAs directly waste researchers' time and money, and an effective way to avoid this result is to select efficient sgRNAs. At present, although there is some sgRNA design software, most of these software design sgRNAs gene by gene and lack a bioinformatics workflow of a design for customizing a genome-wide sgRNA library. Therefore, to provide a system for constructing a customized genome-wide sgRNA library to obtain high-quality genome-wide sgRNAs has important scientific research values and application prospects.

### SUMMARY

In view of the deficiencies of the existing art and practical requirements, the present application provides a system for constructing a genome-wide sgRNA library and a use thereof. By constructing three modules in the system, optimizing details and processes in the modules, and adopting multiple design criteria and screening principles, the genome-wide sgRNA library is finally constructed. The system and method are concise and efficient, and the obtained library has a high quality and good activity, and is convenient for applications in gene editing researches.

To achieve the object, the present application adopts technical solutions described below.

In a first aspect, the present application provides a system for constructing an sgRNA library. The system includes an input module, an sgRNA design module and an sgRNA filtering module.
(1) The input module is configured to download genomic sequences and annotation files from a database, and extract a CDS sequence as an input target sequence.
   This module is configured to prepare input data for the sgRNA design module. The genomic sequences and the annotation files are downloaded from Ensembl or NCBI; position information of a CDS region of each gene is acquired by analyzing the annotation files; and finally, CDS sequences of all genes are extracted from genomic sequence files according to the position information of the CDS region of the each gene and stored in a fasta file as the input target sequence of the sgRNA design module.
(2) The sgRNA design module is configured to select candidate sgRNAs on a sense strand and an antisense strand of the target sequence according to a set parameter, perform a genome-wide sequence alignment, and evaluate off-target rates and grade sgRNAs according to a specified number of allowed mismatches, where 20 nt + NGG is selected as a candidate sgRNA on the sense strand and GGN + 20 nt is selected as the candidate sgRNA on the antisense strand.
   This module designs the sgRNAs on the CDS sequences of all genes according to the following process: 1. finding all sequences meeting conditions on each input target sequence as the candidate sgRNAs according to the set parameter such as a PAM sequence, a sequence length, GC content, a single or double strand mode; 2. the number of allowed mismatches is specified, and the genome-wide sequence alignment is performed on all the candidate sgRNAs; 3. the off-target rates are evaluated according to a number of mismatches and a mismatch position, and qualities of the sgRNAs are graded, according to the off-target rates, as Best, Low_Risk, Moderate_Risk and High_Risk (off-target risk gradients).
(3) The sgRNA filtering module is configured to screen evaluated and graded sgRNAs according to the following criteria: removing an sgRNA including 4 or more consecutive bases, ensuring that sgRNAs have no overlap, and ensuring that the sgRNAs are evenly distributed on a CDS as much as possible.

Preferably, a selection criterion of the target sequence in step (1) includes that a CDS region is selected as the target sequence for a protein-encoding gene and an exon region is selected as the target sequence for a non-protein-encoding gene.

Preferably, the parameter in step (2) includes the PAM sequence, the sequence length, the GC content, the number of allowed mismatches and a single/double-strand mode.

Preferably, the number of allowed mismatches in step (2) is 3 to 6, for example, may be 3, 4, 5 or 6, preferably 5.

Preferably, off-target rate evaluation criteria in step (2) are described below.
(a) An sgRNA capable of being accurately aligned to a plurality of sites in a genome is filtered out.
(b) An sgRNA that is only aligned to a position corresponding to the sgRNA in the genome is Best.
(c) For other sgRNAs, a penalty point is gradually decreased according to the mismatch position of 5'->3', and the other sgRNAs are comprehensively scored in conjunction with the number of mismatches, where a larger penalty point corresponds to a higher risk.

Preferably, grading levels in step (2) include four levels: best, low-risk, moderate-risk and high-risk.

Preferably, screening criteria in step (3) further include any one or a combination of at least two of: selecting at most 6 sgRNAs for each target sequence, reserving only a best sgRNA and a low-risk sgRNA, ensuring that a selected sgRNA covers different transcripts of a gene as much as possible, a plurality of sgRNAs of each gene being targeted to different positions of the each gene as much as possible, and GC content of 20% to 80%, for example, may be a combination of selecting at most 6 sgRNAs for each target sequence and reserving only the best sgRNA and the low-risk sgRNA, a combination of ensuring that the selected sgRNA covers the different transcripts of the gene as much as possible and the plurality of sgRNAs of each gene being targeted to the different positions of the each gene as much as possible, a combination of selecting at most 6 sgRNAs for each target sequence, reserving only the best sgRNA and the low-risk sgRNA, ensuring that the selected sgRNA covers the different transcripts of the gene as much as possible, the plurality of sgRNAs of each gene being targeted to the different positions of the each gene as much as possible, and the GC content of 20% to 80%, preferably, the combination of selecting at most 6 sgRNAs for each target sequence, reserving only the best sgRNA and the low-risk sgRNA, ensuring that the selected sgRNA covers the different transcripts of the gene as much as possible, the plurality of sgRNAs of each gene being targeted to the different positions of the each gene as much as possible, and the GC content of 20% to 80%.

In a second aspect, the present application provides a method for constructing an sgRNA library by using the system described in the first aspect. The method includes the following steps:
(1) selecting a target sequence: downloading genomic sequences and annotation files from a database, and extracting a CDS sequence as an input target sequence;
(2) designing sgRNAs: selecting candidate sgRNAs on a sense strand and an antisense strand of the target sequence according to a set parameter, performing a genome-wide sequence alignment, and evaluating off-target rates and grading sgRNAs according to a specified number of allowed mismatches;
   where 20 nt + NGG is selected as a candidate sgRNA on the sense strand and GGN + 20 nt is selected as the candidate sgRNA on the antisense strand;
(3) screening the sgRNAs: screening evaluated and graded sgRNAs according to the following criteria: removing an sgRNA including 4 or more consecutive bases, ensuring that sgRNAs have no overlap, and ensuring that the sgRNAs are evenly distributed on a CDS as much as possible.

Preferably, a selection criterion of the target sequence in step (1) includes that a CDS region is selected as the target sequence for a protein-encoding gene and an exon region is selected as the target sequence for a non-protein-encoding gene.

Preferably, the parameter in step (2) includes a PAM sequence, a sequence length, GC content, a number of allowed mismatches and a single/double-strand mode.

Preferably, the number of allowed mismatches in step (2) is 3 to 6, for example, may be 3, 4, 5 or 6, preferably 5.

Preferably, off-target rate evaluation criteria in step (2) are described below.
(a) An sgRNA capable of being accurately aligned to a plurality of sites in a genome is filtered out.
(b) An sgRNA that is only aligned to a position corresponding to the sgRNA in the genome is Best.
(c) For other sgRNAs, a penalty point is gradually decreased according to a mismatch position of 5'->3', and the other sgRNAs are comprehensively scored in conjunction with a number of mismatches, where a larger penalty point corresponds to a higher risk.

Preferably, levels for the grading in step (2) include four levels: best, low-risk, moderate-risk and high-risk.

As a preferred technical method, the present application provides a method for constructing an sgRNA library by using the system described in the first aspect. The method specifically includes steps described below.
(1) A target sequence is selected: genomic sequences and annotation files are downloaded from a database, and a CDS sequence is extracted as an input target sequence.
   A CDS region is selected as the target sequence for a protein-encoding gene to design the sgRNAs. If a gene has multiple transcripts, all CDS sequences of the transcripts are used as the target sequence. A gene with only a single transcript uses all CDS regions as the target sequence. An exon region is used as the target sequence for a non- protein-encoding gene.
   The genomic sequences and the annotation files are downloaded from Ensemble or NCBI; position information of a CDS region of each gene is acquired by analyzing the annotation files; and finally, CDS sequences of all genes are extracted from genomic sequence files according to the position information of the CDS region of the each gene and stored in a fasta file as the input target sequence of an sgRNA design module.
(2) sgRNAs are designed: candidate sgRNAs are selected on a sense strand and an antisense strand of the target sequence according to a set parameter including a PAM sequence, a sequence length, GC content and a single/double-strand mode, where 20 nt + NGG is selected as a candidate sgRNA on the sense strand and GGN + 20 nt is selected as the candidate sgRNA on the antisense strand; a genome-wide sequence alignment is performed, where a mismatch farther from the PAM sequence (NGG or GGN) more easily results in an off-target; off-target rates are evaluated according to a number of allowed mismatches and the sgRNAs are graded as best, low-risk, moderate-risk and high-risk (off-target risk gradients); and sgRNAs are selected, where moderate-risk and high-risk sgRNAs are removed, a Best sgRNA is preferably selected, and a low-risk sgRNA is secondly selected.
   Off-target rate evaluation criteria are described below.
   (a) An sgRNA capable of being accurately aligned to a plurality of sites in a genome is filtered out.
   (b) An sgRNA that is only aligned to a position corresponding to the sgRNA in the genome is Best.
   (c) For other sgRNAs, a penalty point is gradually decreased according to a mismatch position of 5'->3', and the other sgRNAs are comprehensively scored in conjunction with a number of mismatches, where a larger penalty point corresponds to a higher risk.
(3) The sgRNAs are filtered: evaluated and graded sgRNAs are screened according to the following criteria: removing an sgRNA including 4 or more consecutive bases, ensuring that sgRNAs have no overlap, ensuring that the sgRNAs are evenly distributed on a CDS as much as possible, selecting at most 6 sgRNAs for each target sequence, reserving only the best sgRNA and the low-risk sgRNA, ensuring that a selected sgRNA covers different transcripts of a gene as much as possible, a plurality of sgRNAs of each gene being targeted to different positions of the each gene as much as possible, and GC content of 20% to 80%.

For the protein-encoding gene, an sgRNA close to a 5' end is preferably selected, and a number of sgRNAs for each CDS is not more than 2. For the non-protein-encoding gene, 4 sgRNAs are designed on an exon sequence of the gene, and the designed sgRNAs have no overlap.

The selected sgRNA should cover different transcripts of a gene as much as possible and be evenly distributed on different CDSs as much as possible, so as to enable the designed sgRNA to ensure that all transcripts of the gene are knocked out, and multiple sgRNAs of each gene are targeted to different positions of the each gene as much as possible to ensure knockout efficiency.

In a third aspect, the present application provides a genome-wide sgRNA library constructed according to the method described in the second aspect.

Compared with the existing art, the present application has the following beneficial effects:
(1) The system for constructing a genome-wide sgRNA library provided by the present application is concise, efficient, and convenient for operations and practical applications. By optimizing processes and details in modules and adjusting design criteria and screening principles of sgRNAs, sgRNAs are designed and filtered comprehensively and specifically for the whole genome, saving time and labor and facilitating promotion and applications.
(2) The sgRNA library obtained by the construction system of the present application is of high quality. Taking a pig as an example, 91.1% of genes in the whole genome are designed to obtain corresponding sgRNAs, and all sgRNAs have activity that can meet requirements of subsequent experiments.

### BRIEF DESCRIPTION OF DRAWINGS

FIG. 1 is a diagram of a working process of a CRISPR gene editor of the present application;
FIG. 2 is a diagram of a system structure of a CRISPR cluster of the present application;
FIG. 3 is a diagram of a principle of a CRISPR/Cas9 targeting system of the present application; and
FIG. 4 is a flowchart for sgRNA construction of the present application.

### DETAILED DESCRIPTION

To further elaborate on the technical means adopted and the effects achieved in the present application, the technical solutions of the present application are further described below with reference to the drawings and specific embodiments, but the present application is not limited to the scope of the embodiments.

### Example 1

A system for constructing a genome-wide sgRNA library is created. The system includes an input module, an sgRNA design module and an sgRNA filtering module.
(1) The input module is configured to download genomic sequences and annotation files from a database, and extract a CDS sequence as an input target sequence.
(2) The sgRNA design module is configured to select candidate sgRNAs on a sense strand and an antisense strand of the target sequence according to a set parameter, perform a genome-wide sequence alignment, and evaluate off-target rates and grade sgRNAs according to a specified number of allowed mismatches.
   20 nt + NGG is selected as a candidate sgRNA on the sense strand and GGN + 20 nt is selected as the candidate sgRNA on the antisense strand.
(3) The sgRNA filtering module is configured to screen evaluated and graded sgRNAs according to the following criteria: removing an sgRNA including 4 or more consecutive bases, ensuring that sgRNAs have no overlap, and ensuring that the sgRNAs are evenly distributed on a CDS as much as possible, selecting at most 6 sgRNAs for each target sequence, reserving only a best sgRNA and a low-risk sgRNA, ensuring that a selected sgRNA covers different transcripts of a gene as much as possible, multiple sgRNAs of each gene being targeted to different positions of the each gene as much as possible, and GC content of 20% to 80%.

### Example 2

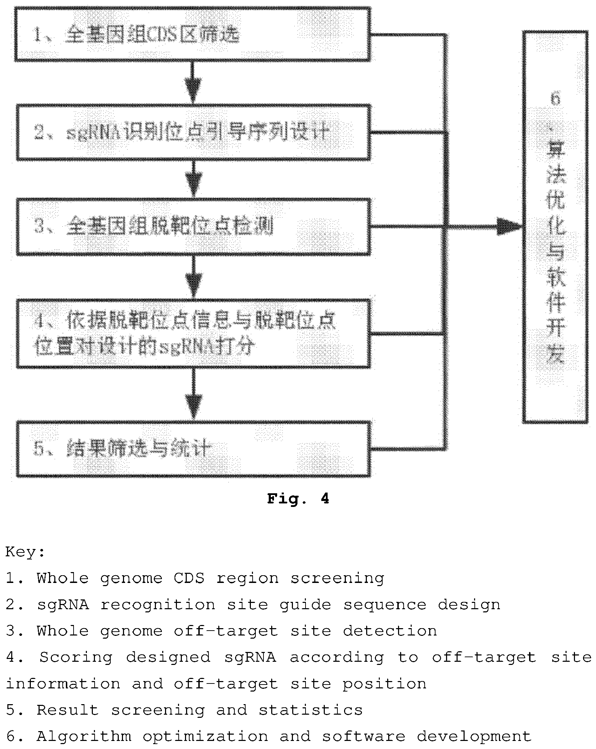

A pig genome-wide sgRNA library is constructed by the system in Example 1. A construction process is shown in FIG. 4. The process sequentially includes screening CDS sequences in the whole genome, selecting candidate sgRNA sequences according to sgRNA recognition sites, detecting off-target sites in the whole genome, scoring designed candidate sgRNAs according to information on the off-target sites and positions of the off-target sites, result screening and design, and algorithm optimization and software development in the whole process. Specific steps are described below.
(1) Genome-wide sequences and annotation files of a pig are downloaded from Ensemble of release90; position information of a CDS region of each gene is acquired by analyzing the annotation files; and finally, CDS sequences of all genes are extracted from genomic sequence files according to the position information of the CDS region of the each gene and stored in a fasta file as an input target sequence of an sgRNA design module. A CDS region is selected as the target sequence for a protein-encoding gene to design sgRNAs. If a gene has multiple transcripts, all CDS sequences of the transcripts are used as the target sequence. A gene with only a single transcript uses all CDS regions as the target sequence. An exon region is used as the target sequence for a non- protein-encoding gene.
(2) Candidate sgRNAs are selected on a sense strand and an antisense strand of the target sequence according to a set parameter including a PAM sequence, a sequence length, GC content and a single/double-strand mode, where 20 nt + NGG is selected as a candidate sgRNA on the sense strand and GGN + 20 nt is selected as the candidate sgRNA on the antisense strand; a genome-wide sequence alignment is performed, where a mismatch farther from the PAM sequence (NGG or GGN) more easily results in an off-target; off-target rates are evaluated according to a number of allowed mismatches specified as 5 and the sgRNAs are graded as best, low-risk, moderate-risk and high-risk (off-target risk gradients); and sgRNAs are selected, where moderate-risk and high-risk sgRNAs are removed, a Best sgRNA is preferably selected, and a low-risk sgRNA is secondly selected.
   Off-target rate evaluation criteria are described below.
   (a) An sgRNA capable of being accurately aligned to multiple sites in a genome is filtered out.
   (b) An sgRNA that is only aligned to a position corresponding to the sgRNA in the genome is Best.
   (c) For other sgRNAs, a penalty point is gradually decreased according to a mismatch position of 5'->3', and the other sgRNAs are comprehensively scored in conjunction with a number of mismatches, where a larger penalty point corresponds to a higher risk.
(3) Evaluated and graded sgRNAs are screened according to the following criteria: removing an sgRNA including 4 or more consecutive bases, ensuring that sgRNAs have no overlap, and ensuring that the sgRNAs are evenly distributed on a CDS as much as possible, selecting at most 6 sgRNAs for each target sequence, reserving only the best sgRNA and the low-risk sgRNA, ensuring that a selected sgRNA covers different transcripts of a gene as much as possible, multiple sgRNAs of each gene being targeted to different positions of the each gene as much as possible, and GC content of 20% to 80%.
   For the protein-encoding gene, an sgRNA close to a 5' end is preferably selected, and a number of sgRNAs for each CDS is not more than 2. For the non-protein-encoding gene, 4 sgRNAs are designed on an exon sequence of the gene, and the designed sgRNAs have no overlap.
(4) General overview of the library: in the constructed pig genome-wide sgRNA library, 20438 genes in total are designed to obtain sgRNAs, of which 17410 genes are designed to obtain 6 sgRNAs and 2828 genes are designed to obtain 1-5 sgRNAs. Results of experiments on sgRNA qualities show that the low-risk sgRNA and above are all high-quality sgRNAs, and sgRNAs in the constructed library all have activity that can meet requirements of subsequent experiments.

The applicant has stated that although the detailed method of the present application is described through the embodiments described above, the present application is not limited to the detailed method described above, which means that implementation of the present application does not necessarily depend on the detailed method described above. It should be apparent to those skilled in the art that any improvements made to the present application, equivalent replacements of various raw materials of the product, the addition of adjuvant ingredients, and the selection of specific manners, etc. in the present application all fall within the protection scope and the scope of disclosure of the present application.

## Claims

1. A system for constructing a genome-wide sgRNA library, comprising:
(1) an input module, which is configured to download genomic sequences and annotation files from a database, and extract a commonly deleted segment (CDS) sequence as an input target sequence;
(2) an sgRNA design module, which is configured to select candidate sgRNAs on a sense strand and an antisense strand of the target sequence according to a set parameter, perform a genome-wide sequence alignment according to a specified number of allowed mismatches, and evaluate off-target rates and grade sgRNAs according to off-target sites and a number of the off-target sites;
wherein 20 nt + NGG is selected as a candidate sgRNA on the sense strand and GGN + 20 nt is selected as the candidate sgRNA on the antisense strand;
(3) an sgRNA filtering module, which is configured to screen evaluated and graded sgRNAs according to the following criteria: removing an sgRNA comprising 4 or more consecutive bases, ensuring that sgRNAs have no overlap, and ensuring that the sgRNAs are evenly distributed on a CDS as much as possible.

2. The system of claim 1, wherein a selection criterion of the target sequence in step (1) comprises that a CDS region is selected as the target sequence for a protein-encoding gene and an exon region is selected as the target sequence for a non-protein-encoding gene.

3. The system of claim 1 or 2, wherein the parameter in step (2) comprises a protospacer adjacent motif (PAM) sequence, a sequence length, guanine-cytosine (GC) content, a single/double-strand mode and a number of mismatches allowed in a genome alignment.

4. The system of any one of claims 1 to 3, wherein the number of allowed mismatches in step (2) is 3 to 6, preferably 5; and
preferably, off-target rate evaluation criteria in step (2) comprise:
(a) filtering out an sgRNA capable of being accurately aligned to a plurality of sites in a genome;
(b) an sgRNA that is only aligned to a position corresponding to the sgRNA in the genome being Best; and
(c) for other sgRNAs, gradually decreasing a penalty point according to a mismatch position of 5'->3', and comprehensively scoring the other sgRNAs in conjunction with a number of mismatches, wherein a larger penalty point corresponds to a higher risk.

5. The system of any one of claims 1 to 4, wherein grading levels in step (2) comprise four levels: best, low-risk, moderate-risk and high-risk.

6. The system of any one of claims 1 to 5, wherein screening criteria in step (3) further comprise any one or a combination of at least two of: selecting at most 6 sgRNAs for each target sequence, reserving only a best sgRNA and a low-risk sgRNA, ensuring that a selected sgRNA covers different transcripts of a gene as much as possible, a plurality of sgRNAs of each gene being targeted to different positions of the each gene as much as possible, and GC content of 20% to 80%, preferably, a combination of selecting at most 6 sgRNAs for each target sequence, reserving only the best sgRNA and the low-risk sgRNA, ensuring that the selected sgRNA covers the different transcripts of the gene as much as possible, the plurality of sgRNAs of each gene being targeted to the different positions of the each gene as much as possible, and the GC content of 20% to 80%.

7. A method for constructing an sgRNA library by using the system of any one of claims 1 to 6, comprising:
(1) selecting a target sequence: downloading genomic sequences and annotation files from a database, and extracting a commonly deleted segment (CDS) sequence as an input target sequence;
(2) designing sgRNAs: selecting candidate sgRNAs on a sense strand and an antisense strand of the target sequence according to a set parameter, performing a genome-wide sequence alignment according to a specified number of allowed mismatches, and evaluating off-target rates and grading sgRNAs according to off-target sites and a number of the off-target sites;
wherein 20 nt + NGG is selected as a candidate sgRNA on the sense strand and GGN + 20 nt is selected as the candidate sgRNA on the antisense strand;
(3) screening the sgRNAs: screening evaluated and graded sgRNAs according to the following criteria: removing an sgRNA comprising 4 or more consecutive bases, ensuring that sgRNAs have no overlap, and ensuring that the sgRNAs are evenly distributed on a CDS as much as possible.

8. The method of claim 7, wherein a selection criterion of the target sequence in step (1) comprises that a CDS region is selected as the target sequence for a protein-encoding gene and an exon region is selected as the target sequence for a non-protein-encoding gene;
preferably, the parameter in step (2) comprises a protospacer adjacent motif (PAM) sequence, a sequence length, guanine-cytosine (GC) content, a single/double-strand mode and a number of mismatches allowed in a genome alignment;
preferably, the number of allowed mismatches in step (2) is 3 to 6, preferably 5;
preferably, off-target rate evaluation criteria in step (2) comprise:
(a) filtering out an sgRNA capable of being accurately aligned to a plurality of sites in a genome;
(b) an sgRNA that is only aligned to a position corresponding to the sgRNA in the genome being Best; and
(c) for other sgRNAs, gradually decreasing a penalty point according to a mismatch position of 5'->3', and comprehensively scoring the other sgRNAs in conjunction with a number of mismatches, wherein a larger penalty point corresponds to a higher risk;
preferably, levels for the grading in step (2) comprise four levels: best, low-risk, moderate-risk and high-risk.

9. A method for constructing an sgRNA library by using the system of any one of claims 1 to 6, specifically comprising:
(1) selecting a target sequence: downloading genomic sequences and annotation files from a database, and extracting a commonly deleted segment (CDS) sequence as an input target sequence;
wherein a selection criterion of the target sequence in step (1) comprises that a CDS region is selected as the target sequence for a protein-encoding gene and an exon region is selected as the target sequence for a non-protein-encoding gene;
(2) designing sgRNAs: selecting candidate sgRNAs on a sense strand and an antisense strand of the target sequence according to a set parameter comprising a protospacer adjacent motif (PAM) sequence, a sequence length, guanine-cytosine (GC) content, a single/double-strand mode and a number of allowed mismatches, performing a genome-wide sequence alignment according to the number of allowed mismatches, and evaluating off-target rates and grading the sgRNAs as best, low-risk, moderate-risk and high-risk (off-target risk gradients) according to a number of mismatches and a mismatch position;
wherein 20 nt + NGG is selected as a candidate sgRNA on the sense strand and GGN + 20 nt is selected as the candidate sgRNA on the antisense strand; and
off-target rate evaluation criteria comprise:
(a) filtering out an sgRNA capable of being accurately aligned to a plurality of sites in a genome;
(b) an sgRNA that is only aligned to a position corresponding to the sgRNA in the genome being Best; and
(c) for other sgRNAs, gradually decreasing a penalty point according to the mismatch position of 5'->3', and comprehensively scoring the other sgRNAs in conjunction with the number of mismatches, wherein a larger penalty point corresponds to a higher risk;
(3) filtering the sgRNAs: screening evaluated and graded sgRNAs according to the following criteria: removing an sgRNA comprising 4 or more consecutive bases, ensuring that sgRNAs have no overlap, ensuring that the sgRNAs are evenly distributed on a CDS as much as possible, selecting at most 6 sgRNAs for each target sequence, reserving only a best sgRNA and a low-risk sgRNA, ensuring that a selected sgRNA covers different transcripts of a gene as much as possible, a plurality of sgRNAs of each gene being targeted to different positions of the each gene as much as possible, and GC content of 20% to 80%.

10. A genome-wide sgRNA library constructed according to the method of claim 9.
